(19) Europäisches Patentamt
European Patent Office
Office européen des brevets

(11) EP 1 224 950 A1

## (12) EUROPEAN PATENT APPLICATION

(43) Date of publication:
24.07.2002 Bulletin 2002/30

(51) Int Cl.7: **A61N 1/36**, A61B 5/103

(21) Application number: 01300493.2

(22) Date of filing: 19.01.2001

(84) Designated Contracting States:
AT BE CH CY DE DK ES FI FR GB GR IE IT LI LU MC NL PT SE TR
Designated Extension States:
AL LT LV MK RO SI

(71) Applicant: Hedgecock, James Lee
Laguna Beach, California 92651 (US)

(72) Inventor: Hedgecock, James Lee
Laguna Beach, California 92651 (US)

(74) Representative: Franks, Robert Benjamin
Franks & Co.
8-10 President Buildings
Saville Street East
Sheffield South Yorkshire S4 7UQ (GB)

### (54) Stair step current CPT measurement method and apparatus

(57) There is disclosed a current perception threshold device using a stepped square wave current for bio-electric stimulation of nerve groups, and a method of use and operation of the device. A step current is preset as a percentage of a maximum peak stimulus current, which flows through a patients skin. As an intensity of the maximum stimulus current is varied, the stair step current varies as a percentage of the intensity of the stimulus current. However, if the stimulus current drops to a user set or preset value of stair step current, the current flowing through the patient's skin is maintained at the set/preset value, and not allowed to go below that value. This avoids the current falling below a cutaneous electrical resistance threshold (CERT) of the patient's skin, at which current conduction ceases.

Fig. 6

EP 1 224 950 A1

## Description

## Field of the Invention

The present invention relates to the field of medical science, and particularly although not exclusively to a method and apparatus utilizing bio-electric stimulation, for medical diagnosis.

## Background to the Invention

It is known to perform diagnostic examination for identifying abnormalities in nerve fibers, by applying an electrical stimulation transcutaneously to a patient.

It is well documented that specific current signal frequencies will selectively stimulate distinct types of nerve fibers, for example 5 Hz selectively stimulates type C nerve fibers, 250 Hz stimulates type A-Delta nerve fibers, and 2000 Hz stimulates type A-Beta nerve fibers. This neuroselectivity of frequencies is exploited by a method employed to measure the lowest level of current intensity a subject can recognize with a transcutaneous electrical stimulus. This method is termed current perception threshold (CPT) diagnosis.

A major problem often encountered during CPT testing is that the cutaneous electrical resistance threshold (CERT), the current signal level required before conduction through skin can occur, may be greater than the current perception threshold, the current at which a patient recognizes that a nerve has been stimulated. In subjects with a greater CERT than CPT the intensity of the diagnosis signal is turned up past the actual CPT without the subject recognizing the stimulus, since the current is not flowing through the skin to the nerve fiber. Once the intensity reaches the CERT and the current begins flowing, then the subject may report a false "high" CPT, which is actually the CERT being reached by the applied signal.

Previously, to a "constant current" mechanism of bio-electric stimulation was developed in the 1950s and refined in the early 1980s. This later refinement is disclosed in US patent 4305402 (Katim). Katim's constant current mechanism monitored a sine wave current and regulated it so that once the CERT had been reached the current was maintained automatically so as to sustain the signal intensity at sufficient level to allow a continuous flow of current, even though a manually operated intensity control may be turned to zero. Thereby, on the next measurement in a serial test at a same skin site on a patient, the current is not required again to breach the CERT, and the actual CPT can thereby be more accurately measured.

Katim's constant current mechanism works best with a sinusoidal wave form current. However, a sinusoidal current is quite difficult for a patient to recognize within a very narrow range of intensities. Due to the wide fluctuation in measurements obtained using a sinusoidal current, measurements must be averaged before meaningful analysis is possible.

A more recognizable stimulus is that of a modulated square wave current. A square wave form current is used in the prior art Medi-DX 7000 CPT diagnostic device of Neuro-DX Associates Incorporated, 445 Dartmoor Street, Laguna Beach, California, 92651-1430. This device enables location and quantification of nerve pathology caused by injury, metabolic, and toxic exposures, and provides a screening method for patients prior to invasive examinations and procedures are undertaken. Results of up to 95% accuracy in the detection and quantification of nerve pathology are achievable.

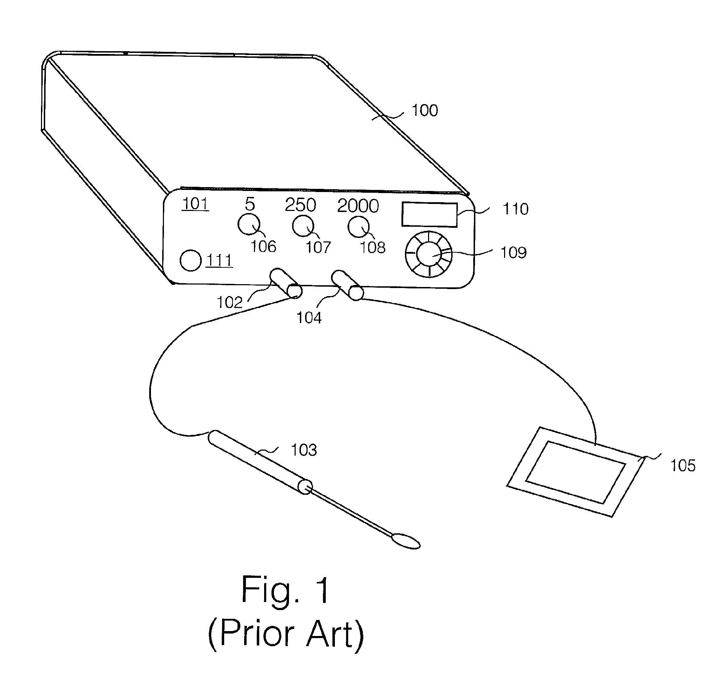

Referring to Fig. 1 herein, there is illustrated schematically in perspective view, the known Medi-DX 7000 current perception threshold diagnostic device. The device comprises a casing 100 containing drive electronics for performing current perception threshold measurements on a patient, the casing having a front panel 101 having a first electrical connector port 102 for connection of a probe device 103; a second electrical connector port 104 for a defuse area electrical contact 105; a set of frequency selector switches 106 - 108 respectively, for selecting test signals having fundamental frequencies corresponding to 5 Hz, 250 Hz and 2 kHz, for testing type C nerves, type A delta nerves, and A-beta nerves respectively; a current intensity control 109 in the form of a rotary dial, having a graduated scale around a circumference of the dial, the rotary dial capable of varying an output current signal in the range 0 to 10 mA between the probe 103 and second electrical contact 105; a liquid crystal display device 110 used to calibrate the current amplitude during manufacture and during after sales service; and an on/off power switch111.

The usage of the device is known in the art, and is as follows:

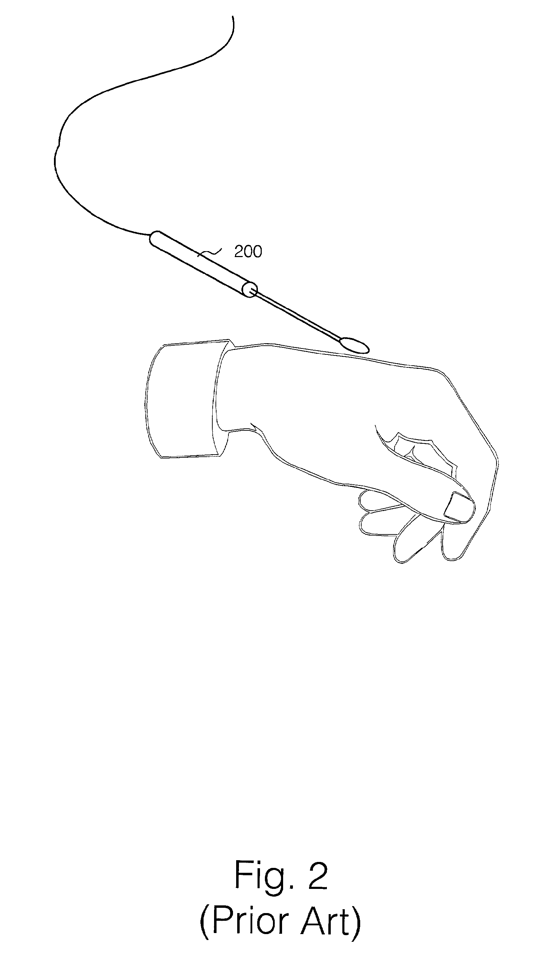

A patient is placed into a relaxed position by a medical personnel. The second electrode contact 105 is placed upon a region of the patient's skin to make electrical contact. The second contact 105 is immersed in saline solution, to improve conductivity between the skin and a wide area contact region of the second contact 105. The probe 103, comprises a gold plated tubular contact 200 capable of receiving a cotton bud 203, which is dipped in saline solution to improve conductivity between the gold contact 200 and a patient's skin as illustrated in Fig. 2 herein. The probe 103 and second electrical contact 105 are placed at various positions around the patient's body, and a square wave electrical signal is passed between the probes through the patient's skin, in order to test various nerves around the patient's body as is known in the art.

To test a particular nerve, the medical operator places the probe and contact at specified positions on the persons body and starting from a zero reading, on the rotary current dial 109 corresponding to zero mean current and gradually increases manually the current by rotating the current control 109, until the patient indicates that a sensation is felt. Due to variations in connection resistance between the cotton bud on the end of the probe 103, and the patient's skin, the medical operator repeats this process 3 or more times for every measurement position, in order to reject spurious readings, and to take a set of readings which are consistent with each other, and which can be used to derive an average reading. Since the medical operator relies upon the patient's perception of sensation due to current, the patient may, either voluntarily or involuntarily, give a misleading indication of when a sensation is felt. For example a patient may, by the intonation of the human medical operator's voice, anticipate when to indicate sensation. Therefore the operator must be careful not to give any indication to the patient of when a sensation could be expected.

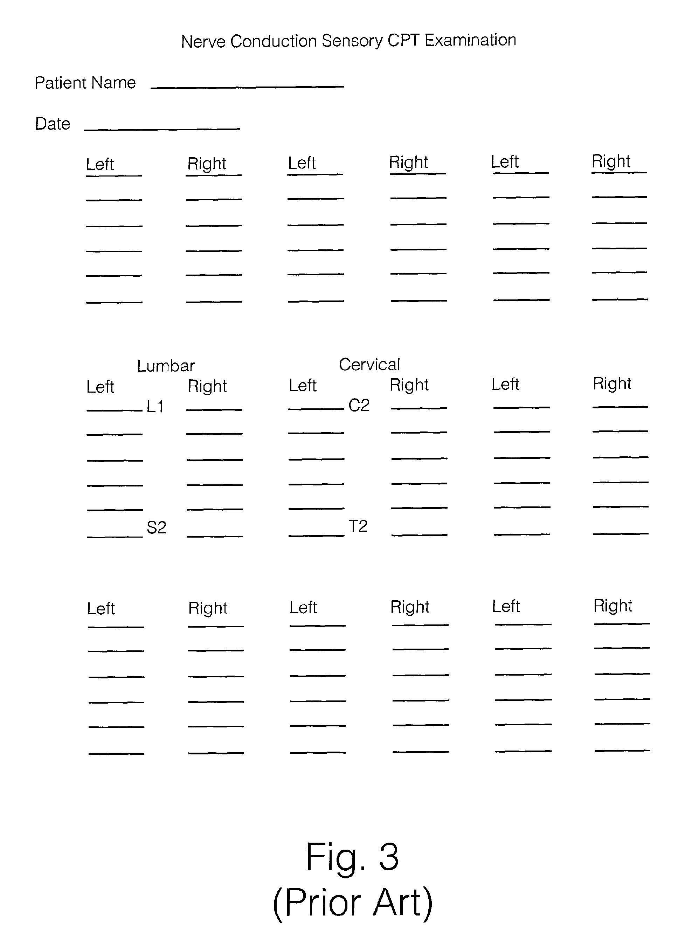

For each nerve tested, the operator manually fills in a record sheet similarly as illustrated in Fig. 3 herein. For example for a cervical test, nerves from the C2 to Thoracic 2 nerve may be tested, on both the left side of the patient's body and the right side of the patient's body and entered onto the record sheet as a current intensity reading on a scale 0 - 100, corresponding to a peak current of 0 to 10 mA. Similarly the medical operator places the probe 103 and second contact 105 on the patient's skin and records readings for the Lumbar L1 to S2 nerves, and other nerve groups as is known in the art.

However, there is a problem in measurement, arising from the electrical conductivity characteristics of a patient's skin. When the probe 103 and second contact 105 are applied to a patient's skin, electrical contact is made via the saline solution, for a current value which has a peak to peak value of a skin conductivity threshold value (CERT), which is determined by the conductive characteristics of the patient's skin. Once the CERT is breached and current is flowing through the skin, provided electrical contact is not lost, and provided the current values does not fall too far below the cutaneous electrical resistance threshold, then current will continue to flow even below the CERT.

However, there is a difficulty in recording readings if the operator returns the rotary current control dial 109 to give a signal too far below the skin's CERT value. Once the current either falls too far below the skin conduction threshold value and current ceases to flow, or if the electrical contact is broken, at a current value below the CERT then the operator must again increase current to exceed the threshold value, before readings can recommence. Additionally, for nerves which respond to currents near the skin conduction threshold value, obtaining accurate readings is made more difficult.

On some patients, the CERT for some skin sites is higher than the current perception level (CPT). In these cases, measurement is difficult because the operator must first breach the CERT, and reduce the current below the CERT, whilst still maintaining current conduction through the skin, in order to test the patients current perception threshold. If at any time the operator reduces the current dial to zero, current conduction will be lost, similarly if the electrical contact between the probe and the skin is broken, then current ceases and measurements must be re-started at the same site. The current value at which current flow stops is characteristic of each individual patient, and is not a known fixed number.

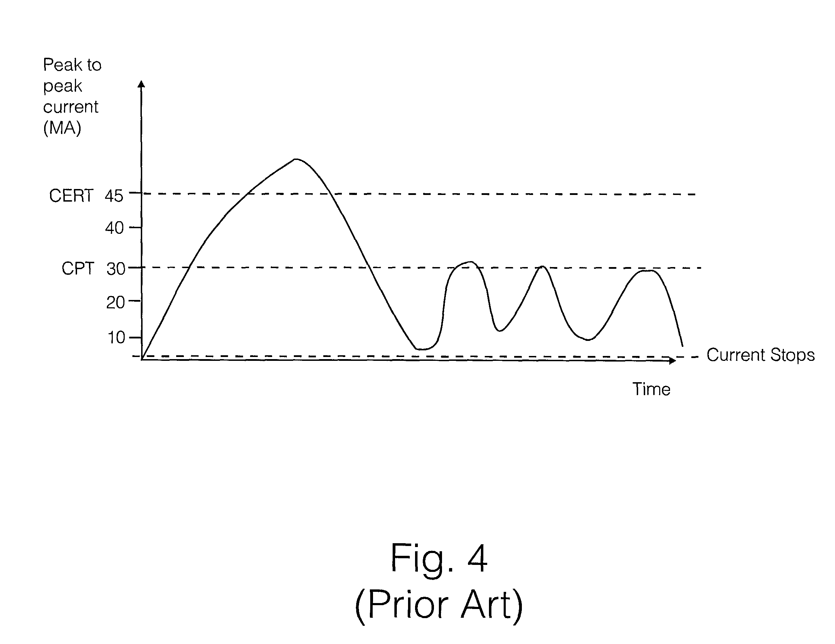

The problem is exemplified, by the plot of peak to peak current versus time shown in Fig. 4 herein which plots an example of the peak to peak current, as controlled by the medical operator, relative to a skin conduction threshold level 401 and a nerve sensitivity threshold level 402.

In this example, the CERT is above the CPT. Initially, the operator turns the current dial from zero up to, for example a reading of 45, at which point the patient indicates that sensation can be felt. This could either be the CERT, or the CPT. At this stage the operator cannot tell which. The operator therefore reduces the current down to a lower reading of 10, and raises the current again slowly. When the dial reads 30, (the level of the CPT) the patient indicates that a sensation is felt, therefore this is likely to be the CPT. However, to verify that, the operator again drops the current to a value of 10, and slowly raises it through the 30 level, at which the patient again indicates a sensation. To verify this a further time, the operator reduces the current back to 10 and raises slowly through 30 at which point the patient verifies a sensation at the current level of 30. In this case, the CERT has a value of 45, and the measured CPT has a value of 30.

The user protocol to deal with this measurement includes:

- At no time can the probe or electrical contact be lifted from the skin sites.
- At no time during the sequence of readings can the intensity be turned to zero (and ideally should not be reduced below a reading of 10 corresponding to 1.0 mA).
- Once the current is turned down, the patient is asked if they continue to feel a stimulus, and if not, then the current is turned up until they feel the stimulus again.
- If an initial higher current measurement is found, then the operator suspects that it is possibly the CERT reading. The operator then turns down the current, but not so far as previously, and asks if the patient continues to feel a stimulus. The operator then turns up the current again until the patient indicates stimulus is felt.
- If the same high reading is noted as previously, then that is the actual CPT and it is a true high reading. Otherwise the initial high reading is a breach of the CERT, and the CPT lies below the initial high reading.

As another example, an operator may initially increase the current to a reading of 45 (4.5 mA) turn down the current to 10 and raise it again for a second reading at 45. The current is then turned down to 20 and a third stimulus reading is recorded at a value of 38. Subsequently the current is turned down to 20 again and a fourth reading is measured at a current of 38 and similarly the fourth or fifth reading also a current is measured at 38. In this case, the CPT is at a value of 38 (3.8 mA) and the CERT (the first two readings) is at a value of 45 (4.5 mA).

If the current is allowed to drop below a critical level at which the current stops (typically between 0 and 10) then current conduction through the skin ceases and the measurement sequence must be started again.

Therefore, to obtain a reliable set of readings, the medical operator must not vary the probe contact to the skin between readings, and ideally should not let the current drop to zero during a set of consecutive readings.

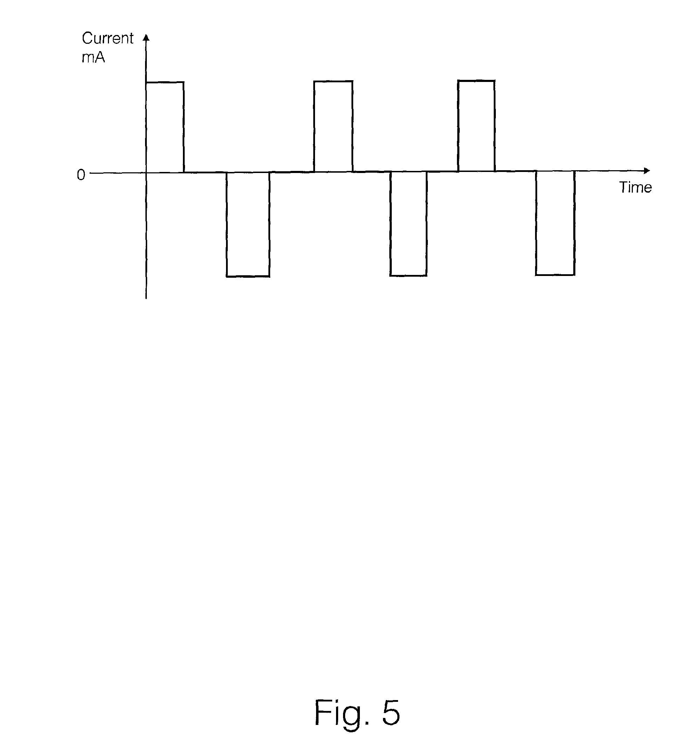

The inventor has realised that a more recognizable stimulus signal than a sine wave or square wave is that of a modulated square wave form as shown in Fig. 5 herein, in which a duty cycle is controlled so as to produce an interval of zero current between an alternating positive and negative square wave cycle. However, it has been noticed that this brief interval of zero current can interrupt the current flow, especially when testing with lower frequencies. This interruption necessitates breaching the CERT again, with a false high CPT measurement.

**Summary of the invention**

A specific implementations of the present invention aim to correct the problem of interruption of stimulus signal current flow by use of a unique current wave form which has not been employed previously in experimental CPT testing, or in any device used for CPT testing. This new wave form is similar to a modulated square wave form current with a zero interval, however instead of an interval between square waves being zero, the current steps down a controllable percentage of a preceding major part of the wave form. A circuit controls this step current as a percentage of a major stimulus current. Therefore current flow is maintained since current does not completely stop flowing through the patient's skin. The step current is maintained at its highest level until a stair step reset operation returns the step current to zero before a new site (nerve) is tested. This mechanism allows any major stimulation wave form to be decreased, but not below a predetermined lower step current value.

One object according to the specific implementations of the present invention is to provide a single wave form which maintains current conduction through skin during variation of a stimulus current signal amplitude.

A second object of specific implementations of the present invention is to improve the usability of a measurement apparatus using bio-electric stimulation.

According to a first aspect of the present invention there is provided a method of diagnostic and therapeutic treatment of a patient comprising the steps of:

attaching a source of electrical signal to a skin region of the patient;

applying an alternating current electrical signal from said source, said signal comprising a stimulation signal element for providing electrical stimulation to said patient, and a step signal element for maintaining a signal flow of said signal through said skin region; and

recording a value of said electrical stimulation signal element at which a nerve stimulation is identified.

According to a second aspect of the present invention there is provided a medical diagnostic apparatus for applying an electric stimulation signal to a patient, said apparatus comprising:

a signal generator circuit for generating an electrical stimulation current signal;

a display device for generating a display of a value of said stimulation current;

a frequency selector circuit, for selecting a frequency of said electrical stimulation signal; and

first and second electrical contacts for making contact of said electrical stimulation current signal with a patients skin, characterised in that.

said electrical stimulation signal is alternating, having a positive cycle and a negative cycle, wherein said signal has a step element, variable as a percentage of a maximum peak level of said signal.

According to a third aspect of the present invention there is provided a method of generating an electrical stimulation current for current perception threshold measurements, said method comprising the steps of:

setting a first amplitude level, of a first portion of a cycle of said current;

setting a second amplitude level over a second portion of said cycle;

wherein said second signal amplitude varies dependent upon said first amplitude level.

## Brief Description of the Drawings

For a better understanding of the invention and to show how the same may be carried into effect, there will now be described by way of example only, specific embodiments, methods and processes according to the present invention with reference to the accompanying drawings in which:

Fig. 1 illustrates schematically the prior art Medi-DX 7000 current perception threshold diagnostic device;

Fig. 2 illustrates schematically placement of a probe contact above a nerve of a patients left arm;

Fig. 3 illustrates schematically one example a record sheet for recording current intensity readings giving rise to responses of nerve stimulation;

Fig. 4 illustrates schematically a trace of peak to peak current amplitude against time for measurement of a current perception threshold level of a patient by a prior art technique;

Fig. 5 illustrates schematically a modulated square wave signal, the use of which in CPT measurement is novel, having an alternating current square wave, interrupted by regions of zero current;

Fig. 6 illustrates schematically a control panel of a novel bio-electric stimulation testing apparatus according to a specific embodiment of the present invention;

Fig. 7 illustrates schematically an electrical stimulation drive wave form generated by the test apparatus of Fig. 6;

Fig. 8 illustrates schematically component signals of the wave form of Fig. 7;

Fig 9 illustrates schematically an example of an output of the bio-electric stimulation test apparatus of Fig. 6, varying over time;

Fig. 10 illustrates schematically a circuit diagram comprising the bio-electric stimulation test apparatus of Fig. 6; and

Fig. 11 illustrates schematically steps for usage of the bio-electric stimulation device for recording measurements of nerve sensitivity.

## Detailed Description of the Best Mode for Carrying Out the Invention

There will now be described by way of example the best mode contemplated by the inventors for carrying out the invention. In the following description numerous specific details are set forth in order to provide a thorough understanding of the present invention. It will be apparent however, to one skilled in the art, that the present invention may be practiced without limitation to these specific details. In other instances, well known methods and structures have not been described in detail so as not to unnecessarily obscure the present invention.

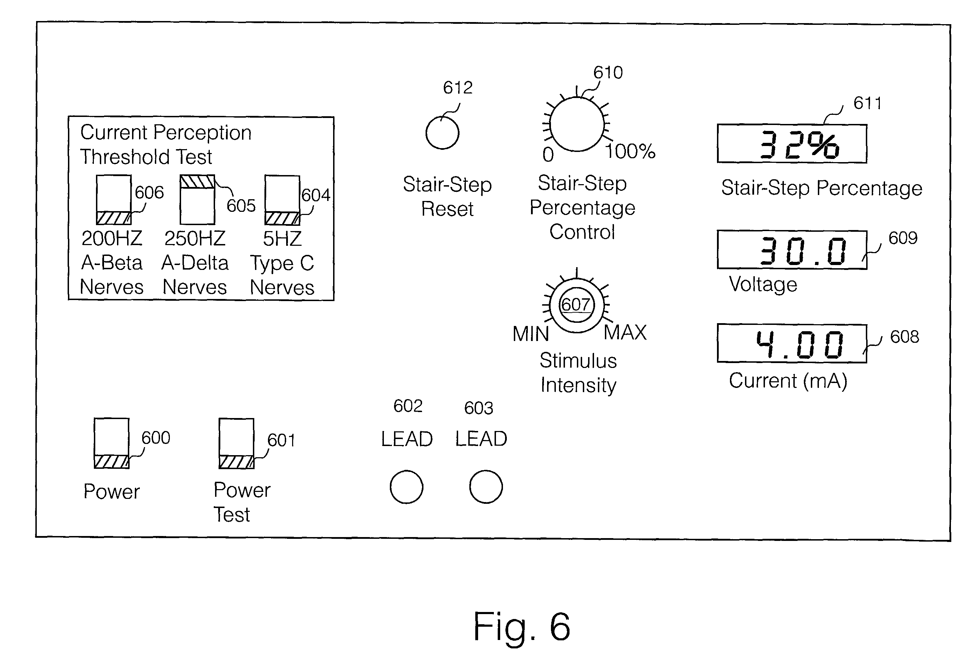

Referring to Fig. 6 herein, there is illustrated a schematically a front panel of a bio-electric stimulation test apparatus according to a specific implementation of the present invention. The control panel comprises an on/off power switch 600; a power test switch 601, for testing the power supply to the device; an output port 602 for a probe device, as herein described with reference to Fig. 1; an output lead 603 for a second electric contact device, as described herein above; a first frequency switch 604 set to a 5 Hz frequency for testing type C nerves; a second frequency switch 605 set to a 250 Hz frequency for testing type A-Delta nerves; a third frequency switch set to 2 kHz, set for testing type A-Beta nerves; a current intensity control dial 607 having a graduated scale from 0 to 100; a first liquid crystal display 608 for displaying a current reading in mA; a second liquid crystal display 609 for displaying a voltage between the probe and second electrical contact; a second rotary current intensity control 610 for controlling a percentage of step current, the second rotary control knob, optionally having a graduated scale of 0 to 100%; a third liquid crystal display 611, configured to display a step current as a percentage of stimulation current; and a step current reset button 612, for resetting the step current to zero.

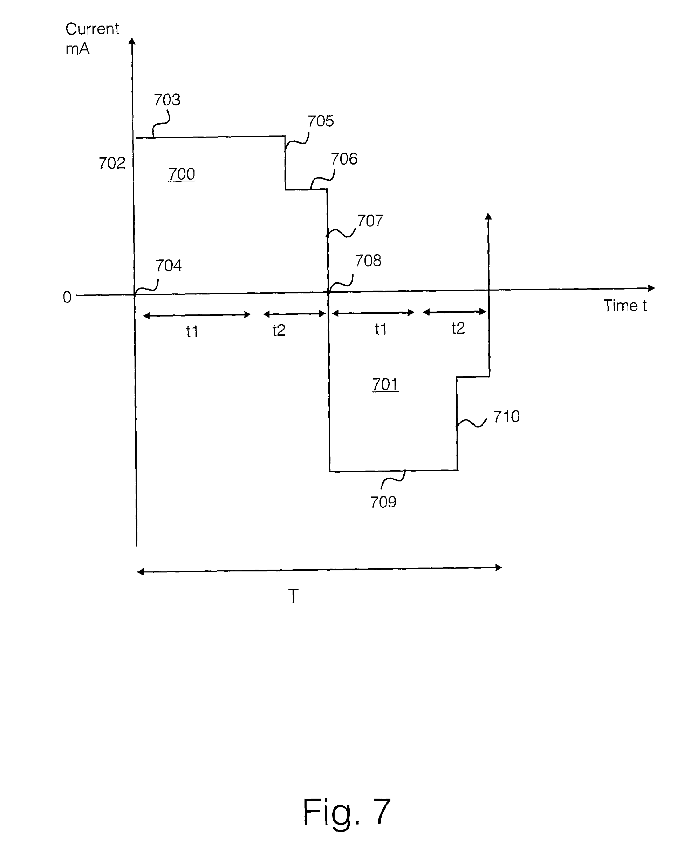

Referring to Fig. 7 herein, there is illustrated a wave form of a drive current generated by the stimulation test apparatus between first and second ports 602, 603 for application between a tip of a probe, and a second electrical contact, through a patients skin. The current wave form is characterised by a positive pulse 700 followed by a negative pulse 701, repeating as an alternating current. One complete cycle consisting of a positive pulse 700 and a negative pulse 701 has a time duration T. The positive pulse 700 has a positive leading edge 702 followed by a constant stimulation current portion 703, followed after a first time period t1 after a positive going zero crossing 704 by a first negative going trailing edge 705, followed by a second constant positive step current portion 706 of duration time t2, followed by a second negative going trailing edge 707 crossing through zero current at negative going zero point 708, at which the current becomes negative; followed by a first negative constant stimulation current portion 709 of duration time t1 after the negative going zero crossing 708, followed by a second positive going trailing edge 710, followed by a constant negative step current portion 711 of duration t2 after the trailing edge 710 which leads back into a first positive going trailing edge 712 of a next cycle.

The ratio of the time t1 to t2 is variable from t1/(t1+t2) = 0% to t1/(t1+t2)= 100%, by adjustment of a potentiometer device or similar.

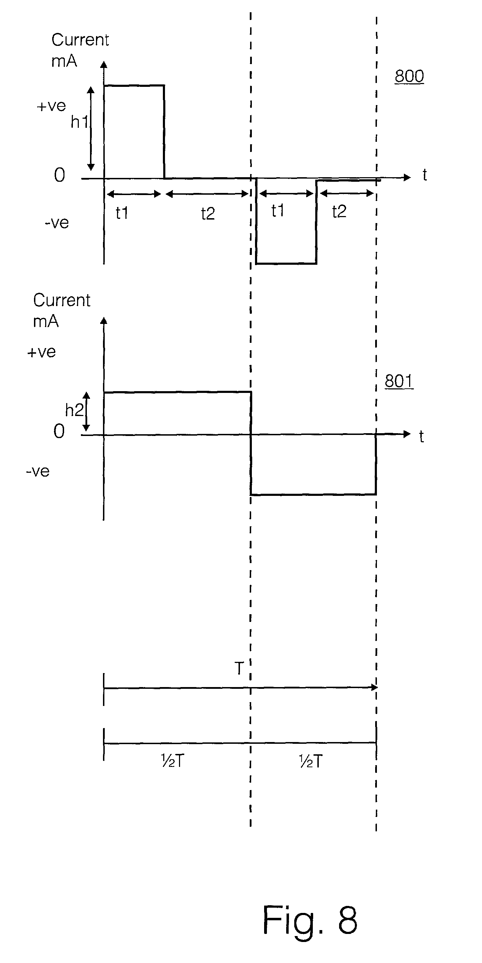

Referring to Fig. 8 herein, the wave form of Fig. 7 can be constructed from a first pulse wave form 800, and a second pulse wave form 801 by adding these two current wave forms. First current wave form has a first square wave of current amplitude of height h1. The second square wave form 801 has current amplitude height h2. The two current amplitudes h2 and h1 are independently variable, by variation of stair step percentage control knob 610, so that the ratio h2/(h1+h2) is continuously variable from 0% to 100%, with a human operator selecting the percentage.

A minimum value of h2 can be preset by a suitable mechanism, such as by adjustment of a potentiometer provided for that purpose.

If a first pulse wave form 800 is used alone, then particularly at low frequencies such as 5Hz, at some sites on some patients, the zero interval between alternating pulses is long enough to stop the flow of current through the patients skin. Using the modulated pulse wave form 800 alone, without the step level (step amplitude height H2 = 0% of first square wave amplitude H1), a typical test may be as follows:

- Operator raises the stimulus amplitude h1 to a first reading of 45 (4.5 mA), at which the patient indicates stimulus.
- Operator turns current level down to 10 and takes a second reading raising the current to 45, at which the patient indicates stimulus.
- Operator turns down the current to 20 and raises again to a level of 45, at which the patient indicates stimulus.
- Operator turns down the current to a level of 30 and raises again to 45 at which the patient indicates stimulus.
- Operator turns down the current to 40 and raises again to a reading of 45 at which the patient indicates stimulus. The level of 45 could be a CERT, and not the CPT. This could be because the current is being interrupted in the period between pulses in the wave form 800, so each time, the CERT must be breached before a patient will indicate sensation.

However, if the step value is applied, at a level of 25% of the stimulus current (h2/(h1 + h2) = 0.25, then the test may run as follows:

- Operator increases maximum current value to a reading of 45 at which point the patient indicates stimulus.
- Operator turns down the current level to 10, but because the step level never goes below 25% of 45 (level of 11.25 corresponding to 1.25 mA, current conduction is maintained, throughout the whole of the duty cycle of the wave form).
- Operator turns up the current through a value of 30, at which point the patient indicates stimulus.
- Operator again reduces the dial to 10 (the step current maintains the real current level at a value of 11.25), and raises again through level 30 at which point the patient indicates a stimulus.
- The cycle is repeated with the operator reducing the dial to 10 again and raising to 30, with the patient indicating a stimulus at level 30.

In this case, the CPT is 30, and the CERT is 45. Because the current is not allowed to drop below a pre-determined level of the step value set as 25% of 45, current flow is always maintained, so the CERT is not breached for a second time before the initial reading of 45.

If, in the above sequence, the level of 45 is continuously indicated by the patient, then the step down percentage can be increased, to for example 50%. This may indicate that the step level has been set too low by the operator and current flow is still being interrupted. However, in the vast majority of cases, a step current level of 25% of the peak stimulus current gives a valid reading.

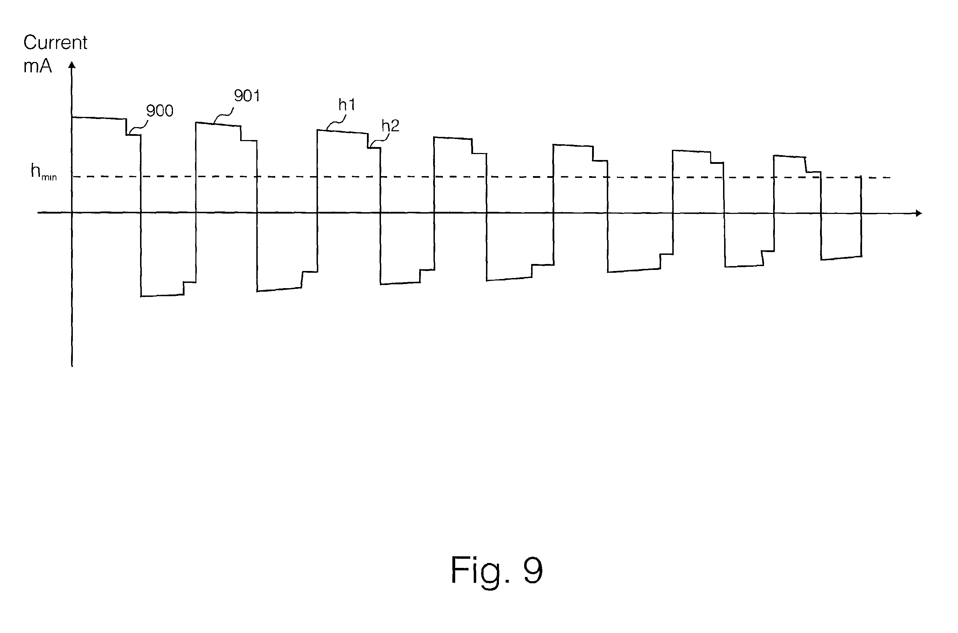

Referring to Fig. 9 herein there is illustrated schematically an example of the wave form changing over time, as a human operator rotates the stimulus current intensity dial 607, reducing the peak stimulus current.

Over time, as the peak current intensity h1 corresponding to the value of first stimulus current value 800 is reduced, because the stair step current value h2, corresponding to the step current square wave form 801 is locked in as a proportion of the stimulus current h1, the step portion of the wave form 800 reduces proportionally with the peak stimulus current according to the relationship.

$$h2 = h1 \times S \text{ for all } h2 > h_{min}$$

$$h2 = h_{min} \text{ for all } h1 \text{ less than or equal to } h_{min}$$

Where S is user a selectable stair step current value as a percentage of stimulus current, in the range 0 to 1.0, and $h_{min}$ is a user selectable or factory preset step current value with magnitude greater than 0.

Therefore as the current is reduced, the maximum stimulus current 901 represented by the upper constant portion of the wave form 704 reduces, and whilst the step current level 900 is above the preset minimum step value $h_{min}$, the step current varies as a proportion of the maximum stimulus current. Where the maximum stimulus current approaches the preset minimum step current $h_{min}$ the peak to peak current amplitude of the signal does not fall below $h_{min}$, but is held at the predetermined minimum step current value $h_{min}$.

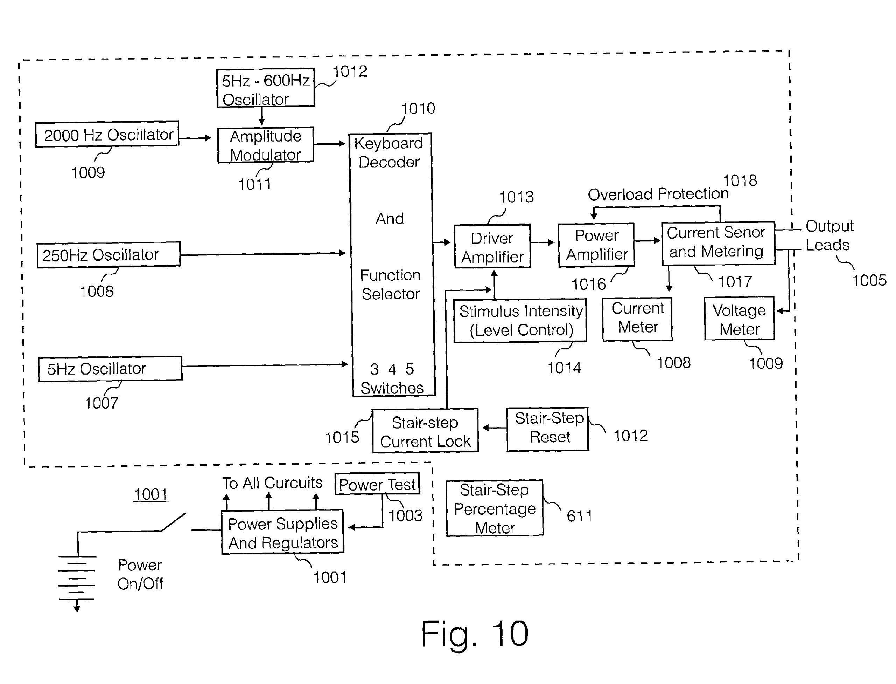

Referring to Fig. 10 herein, there is illustrated schematically a circuit diagram for generating the current wave form of Fig. 7. The circuit may be implemented as discrete components, on a circuit board, or as a dedicated chip, for example an application specific integrated circuit (ASIC), comprising analogue and/or digital components, or by a microprocessor, as will be understood by those skilled in the art. The circuit comprises a power supply 1000, being either a battery power supply or a mains voltage derived power supply as is known in the art; a power off/on switch 1001; set of power supplies and regulators providing power to other components; a power test switch 1003 for testing the power supply; and a wave form generation circuit 1004, supplying the wave form to first and second output leads 1005, 1006 respectively corresponding to first and second output ports 602, 603 in Fig.6.

The wave form generator circuit 1004 comprises a plurality of oscillators 1007-1009 set to oscillation frequencies of 5 Hz, 250 Hz, 2000 Hz respectively; a keyboard decoder and function selector 1010 containing frequency switches 604-606 as described herein before; an amplitude modulator 1011 between the 2000 Hz oscillator 1009 and the keyboard decoder and function selector 1010, the amplitude modulator having an input from a 5 Hz to 600 Hz oscillator 1012; a driver amplifier 1013 receiving an input from the keyboard decoder and function decoder 1010 which selects a wave form type for amplification; a stimulus current intensity level control 1014; a stair step current lock circuit 1015 for locking the step current as a percentage of the stimulation current and setting the minimum step current $h_{min}$; a stair step reset circuit, activated by stair step reset switch 612; a driver amplifier 1013 being driven by the stair step current lock circuit 1015 and stimulus intensity level control 1014; a power amplifier 1016 receiving an output from the driver amplifier 1013 for amplification; and a current sensor and metering device 1017 providing a final output to the output leads 1005, 1006 and applying an overload protection by feedback loop 1018, the current sensor and metering stage 1017 having the current meter 608 and voltage meter 609 monitoring the current and voltage at the output leads 1005 1006.

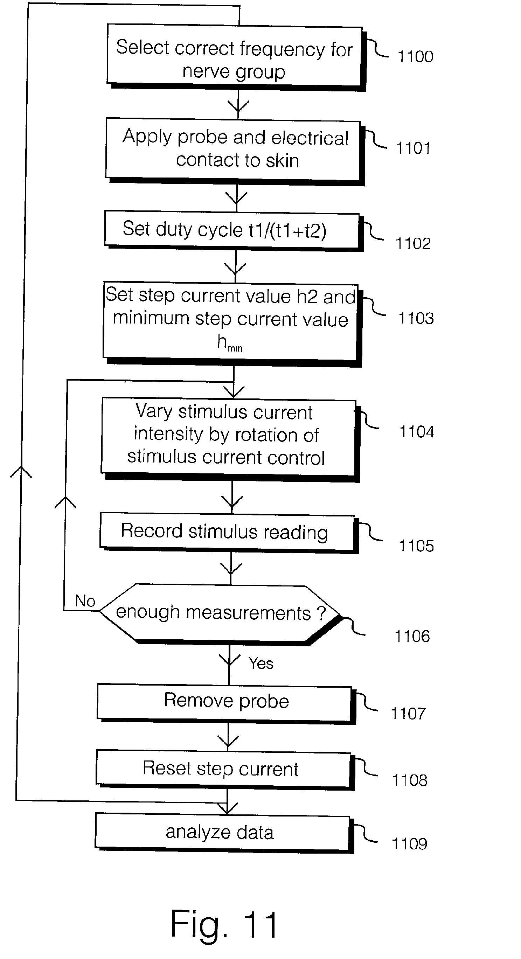

Referring to Fig. 11 herein, there is illustrated schematically a method of use of the stimulation test apparatus. In use, a medical operator applies a probe device and the second electrical contact to a patient's body at the appropriate points, selected for different nerve groups as is known by those skilled in the art. In step 1100, the operator selects a frequency of the signal, either 5Hz, 250Hz or 2kHz in the best mode. In step 1101, the operator applies the probe and electrical contact to the patients skin. In step 1102, the operator sets the relative time duration's of the stimulus current and step current, within the cycle duration T of the wave form. In the best mode herein favorable results have been found with a ratio t1 as 65% of (t1 + t2), in the 250 Hz frequency range, although good performance is found with t2 in the range 25% to 45% of O.S.T. In step 1103, the operator sets the step current value for the set of readings to be taken by rotating the stair step percentage control dial 610 and monitoring visually the reading on the stair step percentage display 611. Steps 1100, 1101 and 1102 need not necessarily be performed in the order shown in Fig. 11. For example the duty cycle, once set may be maintained for different readings, and similarly setting of the step current value h2 as a proportion of the stimulation current value h1. In step 1104 the operator continues to take the first measurement by varying the stimulus current intensity by rotation of the stimulus intensity knob 607, and monitoring the stimulus current display 608. When the patient indicates that a stimulus is felt on the nerve, then in step 1105 the operator records manually by writing down the stimulus intensity currently displayed, onto a record sheet. The steps 1104, 1105 are repeated until in step 1106, enough measurements are recorded for that measurement site. In step 1107, the probe is removed from the patients skin, and in step 1108 the step current is reset to zero, ready for the next set of measurements. In step 1109, the data can be analyzed, having been recorded on a record sheet.

In step 1102, the human operator must first find a suitable value of minimum value stair step current value $h_{min}$, by raising the stair step current to a point where the current display 608 displays an actual current flowing between the electrodes. Since the current is flowing, the human operator knows that the current is above the CERT, and can then reduce the value of the minimum value of stair step current h2 to a minimum value, where current still flows. Having established that current is flowing through the skin, above the CERT, the human operator proceeds to steps 1104, 1105 to rotate the stimulus current intensity dial, varying the height h1 and taking the readings as in step 1105.

In step 1104, because the stair step current value h2 is automatically varied as the stimulus current intensity value h1 is varied and cannot go below a preset value $h_{min}$, then the human operator can return the stimulus intensity dial below the CERT, without losing electrical conductivity through the skin, which is maintained by the minimum value $h_{min}$ of the stair step current value.

**Claims**

1. A method of diagnostic and therapeutic treatment of a patient comprising the steps of:

   attaching a source of electrical signal to a skin region of the patient;

   applying an alternating current electrical signal from said source, said signal comprising a stimulation signal element for providing electrical stimulation to said patient, and a step signal element for maintaining a signal flow of said signal through said skin region; and

recording a value of said electrical stimulation signal element at which a nerve stimulation is identified.

2. The method as claimed in claim 1, further comprising the steps of;
   setting an amplitude of said step signal element dependent upon an amplitude of said stimulation signal element.

3. The method as claimed in claim 2, wherein said amplitude of said step element is set as a fixed percentage of an amplitude of said stimulation signal element.

4. The method as claimed in any one of claims 1 to 3, comprising the steps of:

   setting a fixed value of step current amplitude, below which a current of said step signal will not be generated.

5. The method as claimed in any one of the preceding claims, further comprising the step of:

   setting a time duration of said step signal element as a percentage of a total cycle duration of a wave form of said electrical stimulation signal.

6. The method as claimed in claim 5, wherein said percentage is variable in the range 0% to 100%;

7. The method as claimed in any one of the preceding claims, wherein said electrical stimulation signal is **characterised by** a wave form having:

   a first leading edge raising to a maximum amplitude;

   a first trailing edge dropping from said maximum amplitude to a step amplitude, said step amplitude applied for a time duration after said first trailing edge; and

   a second trailing edge transition reducing said amplitude from said step level, to a zero amplitude.

8. The method as claimed in any one of claims 1 to 7, comprising the step of:

varying a time duration of said step signal element relative to said stimulation element, within a cycle of said electrical stimulation signal.

9. The method as claimed in claim 8, wherein a situation of said step signal element is in the range 25% to 45% of a positive or negative cycle of said signal.

10. A medical diagnostic apparatus for applying an electric stimulation signal to a patient, said apparatus comprising:

    a signal generator circuit for generating an electrical stimulation current signal;

    a display device for generating a display of a value of said stimulation current;

    a frequency selector circuit, for selecting a frequency of said electrical stimulation signal; and

    first and second electrical contacts for making contact of said electrical stimulation current signal with a patients skin, **characterised in that**.

    said electrical stimulation signal is alternating, having a positive cycle and a negative cycle, wherein said signal has a step element, variable as a percentage of a maximum peak level of said signal.

11. The device as claimed in claim 10, comprising a voltage display, displaying a voltage of said electrical stimulation signal.

12. The device as claimed in claim 10 or 11, comprising a step signal percentage display, for displaying an amplitude of said step element of said signal, relative to a peak current amplitude of said signal.

13. The device as claimed in any one of claims 10 to 12, further comprising a step value percentage control device, capable of controlling a value of said step level of said electrical stimulation signal relative to a peak level of said signal.

14. The device as claimed in claim 13, wherein said step percentage control is capable of varying a value of said step element substantially continuously between 0% and 100% of said peak level of said signal.

15. The device as claimed in any one of claims 10 to 14, further comprising a step value reset button, for resetting said step value after a series of measurements.

16. The device as claimed in any one of claims 10 to 15, wherein said current generator circuit is capable of setting a minimum current value of said electrical stimulation signal.

**17.** A method of generating an electrical stimulation current for current perception threshold measurements, said method comprising the steps of:

setting a first amplitude level, of a first portion of a cycle of said current;

setting a second amplitude level over a second portion of said cycle;

wherein said second signal amplitude varies dependent upon said first amplitude level.

**18.** The method as claimed in claim 17, further comprising the step of:

setting said second amplitude portion to be maintained at or above a predetermined minimum amplitude value.

**19.** The method as claimed in 17 or 18, wherein said second amplitude level is set to vary as a percentage of said first amplitude level.

**20.** The method as claimed in any one of claims 17 to 19, wherein a first portion of said first amplitude level is variable between 0 and 100% of a cycle duration of said current.

**21.** The method as claimed in any one of claims 17 to 20, wherein a duration of said second portion is variable between 0 and 100% of a duration of said cycle.

Fig. 1
(Prior Art)

Fig. 2
(Prior Art)

Nerve Conduction Sensory CPT Examination

Patient Name  _____

Date  _____

| Left | Right | Left | Right | Left | Right |
|------|-------|------|-------|------|-------|
| ___ | ___ | ___ | ___ | ___ | ___ |
| ___ | ___ | ___ | ___ | ___ | ___ |
| ___ | ___ | ___ | ___ | ___ | ___ |
| ___ | ___ | ___ | ___ | ___ | ___ |
| ___ | ___ | ___ | ___ | ___ | ___ |

|  | Lumbar |  | Cervical |  |  |
|------|-------|------|-------|------|-------|
| Left | Right | Left | Right | Left | Right |
| ___ L1 | ___ | ___ C2 | ___ | ___ | ___ |
| ___ | ___ | ___ | ___ | ___ | ___ |
| ___ | ___ | ___ | ___ | ___ | ___ |
| ___ | ___ | ___ | ___ | ___ | ___ |
| ___ | ___ | ___ | ___ | ___ | ___ |
| ___ S2 | ___ | ___ T2 | ___ | ___ | ___ |

| Left | Right | Left | Right | Left | Right |
|------|-------|------|-------|------|-------|
| ___ | ___ | ___ | ___ | ___ | ___ |
| ___ | ___ | ___ | ___ | ___ | ___ |
| ___ | ___ | ___ | ___ | ___ | ___ |
| ___ | ___ | ___ | ___ | ___ | ___ |
| ___ | ___ | ___ | ___ | ___ | ___ |
| ___ | ___ | ___ | ___ | ___ | ___ |

# Fig. 3
# (Prior Art)

EP 1 224 950 A1

Fig. 4
(Prior Art)

Fig. 5

Fig. 6

Fig. 7

Fig. 8

Fig. 9

Fig. 10

Select correct frequency for nerve group — 1100

Apply probe and electrical contact to skin — 1101

Set duty cycle t1/(t1+t2) — 1102

Set step current value h2 and minimum step current value $h_{min}$ — 1103

Vary stimulus current intensity by rotation of stimulus current control — 1104

Record stimulus reading — 1105

enough measurements ? — 1106
No
Yes

Remove probe — 1107

Reset step current — 1108

analyze data — 1109

Fig. 11

**European Patent Office**

# PARTIAL EUROPEAN SEARCH REPORT

which under Rule 45 of the European Patent Convention shall be considered, for the purposes of subsequent proceedings, as the European search report

**Application Number**

EP 01 30 0493

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 029 090 A (HERBST EWA) 22 February 2000 (2000-02-22) * the whole document * in particular * abstract * | 10,11 | A61N1/36 A61B5/103 |
| Y | | 12-16 | |
| X | GB 2 123 698 A (BIOSTIM INC) 8 February 1984 (1984-02-08) * the whole document * in particular * abstract; figure 7 * | 10,11 | |
| Y | US 4 690 145 A (KING-SMITH ERIC A  ET AL) 1 September 1987 (1987-09-01) * the whole document * | 12-16 | |
| A | US 5 020 542 A (ROSSMANN CHARLES  ET AL) 4 June 1991 (1991-06-04) * the whole document * | 10-16 | |
| A | US 5 797 854 A (HEDGECOCK JAMES L) 25 August 1998 (1998-08-25) * the whole document * | 10-12 | TECHNICAL FIELDS SEARCHED (Int.Cl.7) A61N A61B |

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

10-16

Claims searched incompletely :

Claims not searched :

1-9,17-21

Reason for the limitation of the search:

Article 52 (4) EPC - Method for treatment of the human or animal body by therapy

| Place of search | Date of completion of the search | Examiner |
|---|---|---|
| THE HAGUE | 29 May 2001 | Ferrigno, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C07)

**European Patent Office**

**PARTIAL EUROPEAN SEARCH REPORT**

Application Number

EP 01 30 0493

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | US 4 646 744 A (CAPEL IFOR D) 3 March 1987 (1987-03-03) * the whole document * ----- | 10 | |
| | | | **TECHNICAL FIELDS SEARCHED** (Int.Cl.7) |

EPO FORM 1503 03.82 (P04C10)

## EP 1 224 950 A1

**ANNEX TO THE EUROPEAN SEARCH REPORT
ON EUROPEAN PATENT APPLICATION NO.**          EP 01 30 0493

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2001

| Patent document cited in search report | | Publication date | Patent family member(s) | | Publication date |
|---|---|---|---|---|---|
| US 6029090 | A | 22-02-2000 | AU 2350799 A<br>WO 9937359 A | | 09-08-1999<br>29-07-1999 |
| GB 2123698 | A | 08-02-1984 | AU 1573183 A<br>DE 3321839 A<br>FR 2528709 A<br>JP 59057668 A | | 22-12-1983<br>22-12-1983<br>23-12-1983<br>03-04-1984 |
| US 4690145 | A | 01-09-1987 | DE 3620220 A<br>GB 2177304 A,B<br>JP 61290964 A | | 18-12-1986<br>21-01-1987<br>20-12-1986 |
| US 5020542 | A | 04-06-1991 | NONE | | |
| US 5797854 | A | 25-08-1998 | NONE | | |
| US 4646744 | A | 03-03-1987 | AU 571723 B<br>AU 4603185 A<br>EP 0238480 A<br>WO 8700063 A | | 21-04-1988<br>30-01-1987<br>30-09-1987<br>15-01-1987 |

EPO FORM P0459

For more details about this annex : see Official Journal of the European Patent Office, No. 12/82

23